(19) Europäisches Patentamt / European Patent Office / Office européen des brevets

(11) **EP 3 115 999 A1**

(12) **EUROPEAN PATENT APPLICATION**

(43) Date of publication:
**11.01.2017 Bulletin 2017/02**

(51) Int Cl.:
**H01F 17/00** (2006.01)      **A61N 2/02** (2006.01)
**A61N 1/40** (2006.01)

(21) Application number: **16178722.1**

(22) Date of filing: **08.07.2016**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **10.07.2015  IT UB20152081**

(71) Applicant: **Thereson S.p.A.**
**20871 Vimercate (MB) (IT)**

(72) Inventors:
• **FERMI, Enrico**
  **20871 Vimercate MB (IT)**
• **GALIMBERTI, Giambattista**
  **20871 Vimercate MB (IT)**

(74) Representative: **Meindl, Tassilo**
**Buzzi, Notaro & Antonielli d'Oulx S.r.l.**
**Via Maria Vittoria, 18**
**10123 Torino (IT)**

(54) **PRODUCTION METHOD OF A DIFFUSER AND CORRESPONDING DIFFUSER**

(57)      Described herein is a process for the production of a diffuser comprising the steps of:
- providing a first substrate (300a) and a second substrate (300b), wherein a respective uniform metal layer (302a, 302b) is set on the first substrate (300a) and the second substrate (300b);
- providing a first solenoid on the first substrate (300a) via a process of etching of the respective metal layer in an electrolytic bath, wherein the first solenoid comprises an external connection terminal and an internal connection terminal;
- providing a second solenoid on the second substrate (300b) via a process of etching of the respective metal layer in an electrolytic bath, wherein the second solenoid comprises an external connection terminal and an internal connection terminal;
- fixing the first substrate (300a) to the second substrate (300b); and
- providing an electrical connection between the internal connection terminal of the first solenoid and the internal connection terminal of the second solenoid.

Fig. 7c

**Description**

Technical field

The present description relates to techniques of production of diffusers designed to be used in systems for therapeutic treatments with electromagnetic waves.

Description of the relevant art

The documents Nos. WO 2012/172504 and PCT/IB2015/054797 describe systems for therapeutic treatments with electromagnetic waves.

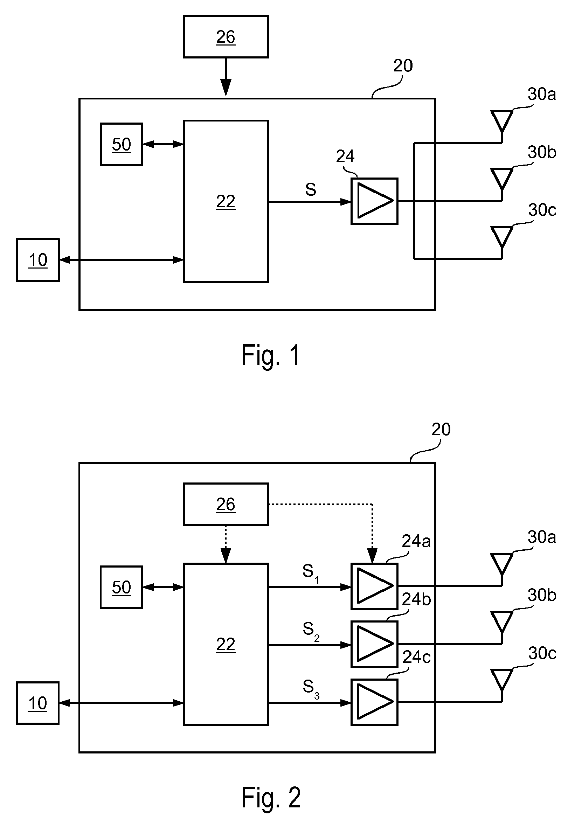

For instance, Figure 1 shows an example of a system for therapeutic treatments.

The system comprises an apparatus 20 that generates at least one driving signal for at least one diffuser 30.

Said apparatus 20 may comprise a control circuit 22 configured for generating a signal S, which is sent through a power amplifier 24 to at least one antenna or diffuser 30. For example, Figure 1 shows three antennas 30a, 30b and 30c.

For instance, for typical therapeutic treatments, the electromagnetic field emitted by a diffuser 30 is frequently between 40 nT and 200 $\mu$T.

In particular, in Figure 1, the control circuit 22 generates just one driving signal S, which is sent by means of an amplifier 24 to one or more diffusers 30; namely, the diffusers are driven with one and the same signal S. In general, also a plurality of amplifiers may be provided; for example, one amplifier could be provided for each diffuser 30, and each amplifier 24 could have a different amplification value set.

Instead, Figure 2 shows a system in which the control circuit 22 generates a plurality of driving signals S. For example, in Figure 2, the control circuit 22 generates three driving signals $S_1$, $S_2$, and $S_3$, which are sent, through respective amplifiers 24a, 24b, and 24, to three diffusers 30a, 30b, 30c; i.e., each diffuser 30 is driven via a respective driving signal S and a respective amplifier 24.

For instance, the treatment system could use at least two driving signals $S_1$ and $S_2$. In this case, the first driving signal $S_1$ can be used for a stimulation with systemic effect. For this purpose, a diffuser is typically used designed to emit electromagnetic radiation towards a system of the person treated, such as the endocrine system, the immune system, the lymphatic system, or the muscular system. Instead, the second driving signal $S_2$ can be used for a stimulation with local effect. For this reason, the first signal $S_1$ typically comprises the characteristic frequencies of the system to be treated, whereas the second signal $S_2$ comprises the characteristic frequencies of the local area to be treated, for example of the tissue or organ.

Figures 1 and 2 moreover show a block 26 that represents a power-supply circuit, such as a DC/DC or AC/DC electronic converter and/or a battery. In general, said power-supply circuit 26 can be incorporated in the apparatus 20 (see Figure 2) or be set at least in part externally (see Figure 1).

Frequently, the system can be connected to an external processing unit 10, such as a PC, which is designed to communicate with the apparatus 20, for example for configuring the apparatus 20 and/or for downloading a treatment protocol from the apparatus 20. Moreover, the apparatus 20 may comprise a user interface 50, including, for example, a plurality of keys and/or state indicators, a touch screen, etc.

Consequently, in the known solutions, the therapeutic electromagnetic fields are administered to the patient via diffusers 30 supplied by a current signal coming from an apparatus 20.

As explained previously, the diffusers 30 are provided for specific applications or treatments and may be in different forms, for example in the form of mats or mattresses, which may be plane or modelled anatomically to reproduce the area where they are applied, or in the form of handpieces.

Typically, said diffusers are provided via plane solenoids that are applied to a support. For example, the diffusers shown in Figures 6 and 7 of the document WO 2012/172504, the content of which is incorporated herein for reference, may be used for this purpose.

Frequently, the above solenoids are produced manually using coiled copper electric wire, or else are based on industrial solutions such as Tesla coils, constituted by rigid copper windings, which can easily undergo deformation, with high risk of breaking and in any case with a high likelihood of introduction of malfunctioning, with a generated variable magnetic field different from the expected one.

Alternative embodiments of traditional flexible or rigid printed-circuit boards cannot be used on account of problems both of maximum size and of high costs. Moreover, the flex-PCB technology is usually reserved to use of low currents, whereas in diffusers for magnetotherapy currents of an intensity higher than 1 A may be used.

Object and summary of the disclosure

The object of the present invention is to provide solutions for the production of diffusers that will overcome one or more of the drawbacks mentioned above.

According to the present invention, the above object is achieved via a process of production of a diffuser having the characteristics that form the subject of Claim 1. The invention also regards a corresponding diffuser.

The claims form an integral part of the teaching provided herein in relation to the invention.

As mentioned previously, the present description provides a solution for the production of diffusers, to be used, for example, in systems for therapeutic treatments with electromagnetic waves.

In various embodiments, the diffuser is provided via etching of a metal material in an electrolytic bath, the so-called wet etching.

In particular, in various embodiments, two independent substrates are provided, where a respective uniform metal layer is set on each substrate. For example, the substrates may be made of a plastic material, such as polyethylene or polyester. Instead, the metal layer may be made of copper or preferably aluminium.

Next, at least one solenoid is provided on the first substrate via a process of etching of the respective metal layer in an electrolytic bath. In particular, the above solenoid is provided via a plane spiral that comprises an external connection terminal and an internal connection terminal. Likewise, at least one solenoid is provided on the second substrate via a process of etching of the respective metal layer in an electrolytic bath. In particular, also this solenoid is provided via a plane spiral that comprises an external connection terminal and an internal connection terminal.

For instance, typically the distance between the metal paths that form the two solenoids is between 0.5 and 5 mm, preferably between 0.8 and 2 mm. Instead, the width of the paths is typically between 1 and 10 mm, preferably between 3 and 7 mm.

In general, the solenoids may be different from one another. However, it is preferable for the solenoids to have substantially the same number of turns. In particular, in some embodiments, the two solenoids may be identical and may be arranged with different angles of rotation.

In various embodiments, the two substrates are then fixed together, and an electrical connection is provided between the internal connection terminal of the two solenoids.

Consequently, according to the present description and as will be described in detail hereinafter, the diffuser comprises solenoids that have been obtained via two independent processing operations, which enables use of metal substrates and layers commonly available on the market, in which the thickness of the metal layer is at most 50 $\mu$m. In general, the solution according to the present description is advantageous when the metal layer has a thickness greater than 30 $\mu$m, preferably between 40 and 80 $\mu$m, preferably between 40 and 60 $\mu$m.

Brief description of the drawings

Embodiments of the present disclosure will now be described in detail with reference to the attached drawings, which are provided purely by way of nonlimiting example and in which:

- Figures 1 and 2 have already been described previously;
- Figures 3 and 4 show an etching process in an electrolytic bath;
- Figures 5 and 6 show a process of production of a diffuser according to the present description; and
- Figures 7 to 9 show some details of embodiments of the process of production according to the present description.

Detailed description of embodiments

In the ensuing description various specific details are illustrated aimed at enabling an in-depth understanding of the embodiments. The embodiments may be provided without one or more of the specific details, or with other methods, components, materials, etc. In other cases, known structures, materials, or operations are not shown or described in detail so that various aspects of the embodiments will not be obscured.

Reference to "an embodiment" or "one embodiment" in the framework of the present description is meant to indicate that a particular configuration, structure, or characteristic described in relation to the embodiment is comprised in at least one embodiment. Hence, phrases such as "in an embodiment", "in one embodiment", or the like, that may be present in different points of the present description do not necessarily refer to one and the same embodiment. Moreover, particular conformations, structures, or characteristics may be combined in any adequate way in one or more embodiments.

The references used herein are provided only for convenience and hence do not define the sphere of protection or the scope of the embodiments.

As mentioned previously, the present description provides solutions for obtaining diffusers that preferably are able to adapt to any anatomic district. For example, these diffusers may be used in the treatment systems described previously.

For instance, in various embodiments, the diffusers provided are flexible, for instance of a type that can be rolled up, and may be of any shape and size. Consequently, there may be provided wearable diffusers with a 3D structure.

In various embodiments, the diffusers are impermeable and made of biocompatible materials.

In various embodiments, the production of the diffusers is based upon standard industrial processes in such a way as to reduce the costs.

In particular, the inventor has noted that a known industrial technique of production of electrical circuits is etching of conductive material in an electrolytic bath, the so-called wet etching.

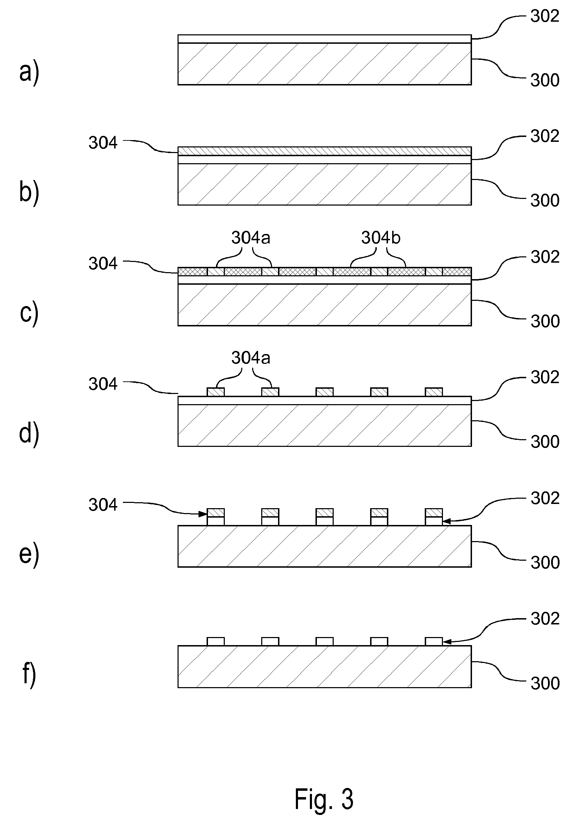

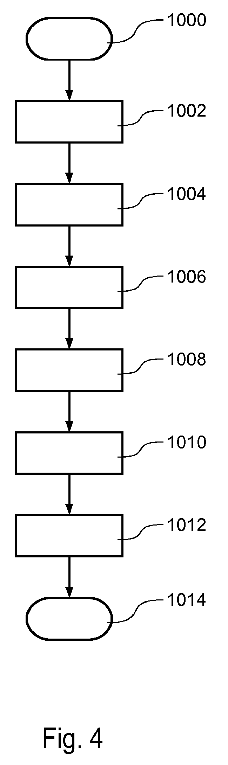

For instance, Figures 3 and 4 illustrate a typical wet-etching process.

After a starting step 1000, in a step 1002 a substrate 300 is provided on which a layer of metal material 302 is deposited in a uniform way (Figure 3a). For example, in various embodiments, the substrate is a layer of plastic material, such as polyethylene or polyester. Instead, the layer of metal material 302 may, for example, be an aluminium or steel layer. For example, already available on the market are sheets of different dimensions, which comprise the substrate and the metal layer, which may have also different thicknesses.

Next, provided on the metal layer 302 is a structured protective layer 304.

For example, in one embodiment, in a step 1004 a photo-resistive material, the so-called "photoresist", such as a photosensitive polymer, is deposited on the metal layer 302 (see Figure 3b). Next, in a step 1006, the structure is irradiated with rays, for example UV rays, which pass through a lithographic mask. Consequently, the mask does not allow passage of the rays in the dark part and the rays change the chemical properties (solubility in an appropriate solvent) of the photoresist in the irradiated areas (see Figure 3c). In the embodiment considered, if the photoresist 304 is positive, in a step 1008 the part of the layer 304 that has been exposed to the rays is chemically removed (see Figure 3d) and the part 304a covered by the mask remains. The person skilled in the branch will appreciate that a negative photoresist may also be used.

Consequently, at the end of the step 1008 a structured protective layer 304 has been provided on the metal layer. In general, the structured protective layer 304 could be provided also in some other way, for example via a protective paint that is printed on the metal layer 302. In this case, a semipermeable serigraphic frame is frequently used, which allows passage of a paint, which is also etch-resistant, only through the holes. Consequently, in this case, the frame represents also a mask that enables passage of the protective paint only where desired.

In the embodiment considered, in a step 1010, the structure is then dipped in an electrolytic bath and, via etching, the conductive material of the layer 302 that is not protected by the structured protective layer 304 is removed (see Figure 3e), i.e., the underlying metal material 302 is removed according to the shape of the structured protective layer 304.

Finally, the structured protective layer 304 can be removed in a step 1012, leaving the structured metal layer (see Figure 3f), and the procedure terminates in a step 1014.

Consequently, a solenoid could be obtained by removing the conductive material where it is not necessary, i.e., "digging" the desired electric circuit in the metal layer 302. In fact, the known wet-etching processes could be used by providing a structured protective layer or etch resist 304 that reproduces the shape of a solenoid.

For example, the technique of the electrolytic bath is used for obtaining heater resistors for various applications, e.g., in the automotive sector. Currently, the heaters are frequently electrical resistors.

The solenoids, necessary for supply of the electromagnetic field, are instead inductors, which must have an extremely low electrical resistance. Consequently, to obtain diffusers there should be provided a set of solenoids that present, instead, a low circuit resistance and desired values of inductance, in order to obtain electromagnetic-field diffusers. The person skilled in the branch will appreciate that the inductance of a plane solenoid can be estimated, for example, with the Wheeler method or the current-sheet method.

Moreover, these diffusers should also have defined mechanical characteristics, for example with reference to flexibility, shape, etc.

To be able to adapt the technology, it is hence necessary to solve primarily the problem of obtaining a low electrical resistance in order to prevent production of heat during use as magnetotherapy diffuser and to prevent having to resort to excessively high supply voltages, which would prevent use thereof for the production of low-voltage medical devices.

In general, apart from the resistivity of the conductive material used, the overall electrical resistance can be reduced by increasing the thickness of the metal layer or the width of the metal paths. However, the magnetic field emitted by a solenoid traversed by electric current is proportional to the intensity of the electric current and to the number of turns. Consequently, the maximum width of the paths can be limited, and it is hence expedient to increase the thickness of the paths.

The inventors have noted that, to be able to use low-voltage supplies (according to the standards currently in force, the voltage should be lower than 50 V) with aluminium metal paths, a thickness of the order of the hundreds of microns, i.e., higher than 100 $\mu$m, in the case of aluminium, would be necessary. Aluminium is particularly advantageous owing to its lower cost as compared to copper and lower environmental impact.

However, it is rare if not impossible to find on the market, and is it difficult to manufacture *ad hoc,* sheets of flexible material, such as polyester or polyethylene, that could be machined with the chemical-etching technique with an aluminium layer deposited thereon of 100 $\mu$m. Moreover, the considerable amount of material to be removed to provide the solenoids would require excessively long operating times, thus increasing the production cost, and would lead to technical problems, such as excessive heat produced during processing with increased risks for the environment and staff, and possible damage to the production equipment. For this reason the thicknesses of the conductive material used in the sheets available on the market are normally at most 50 $\mu$m. However, as explained previously, this thickness is not sufficient for producing diffusers used for treatments with electromagnetic waves of the type described previously.

For this reason, in various embodiments, the solenoids are implemented via two turns that are provided on respective substrates and are electrically connected together, maintaining the circuit continuity and direction of flow of the electric current in order to reduce the overall electrical resistance and consequently the supply power necessary to enable diffusion of a uniform electromagnetic field and to enable use of electrical supply sources also of a portable type (rechargeable batteries).

Consequently, in various embodiments, the thickness of the metal layer is greater than 30 $\mu$m, preferably between 40 and 80 $\mu$m, and typically between 40 and 60 $\mu$m.

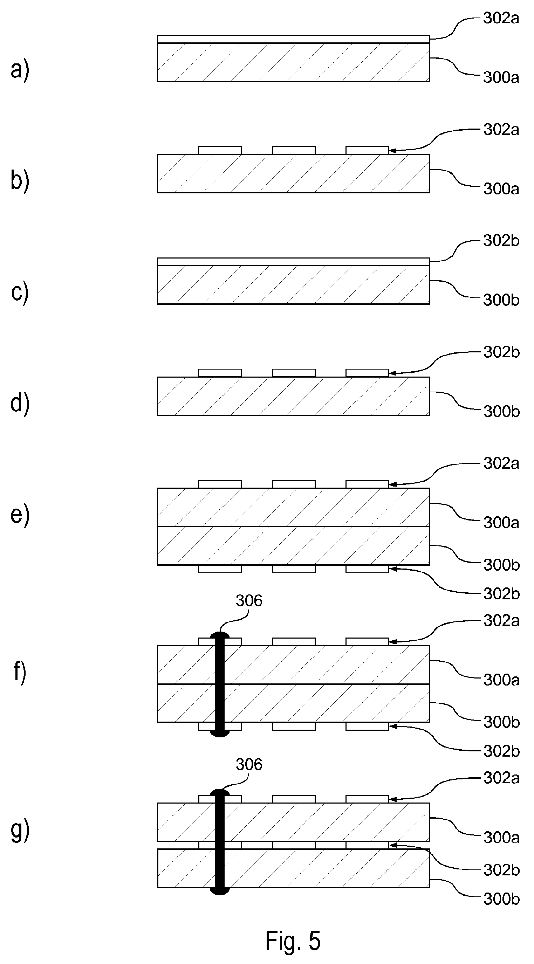

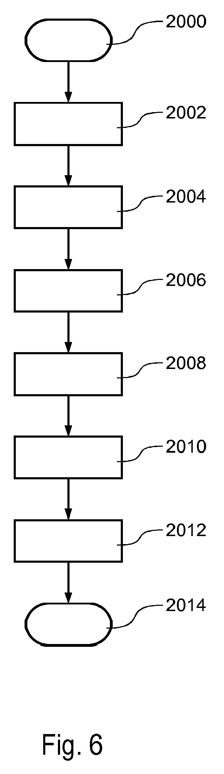

For example, Figures 5 and 6 illustrate a possible embodiment, in which two substrates 300a and 300b with respective metal structures 302a and 302b are provided.

In particular, after a starting step 2000, in a step 2002 a first substrate 300a is provided, deposited in a uniform way on which is a first layer of metal material 302a (see Figure 5a).

In a step 2004, a wet-etching process is used (steps 1000-1013 described with reference to Figures 3 and 4) to provide at least one solenoid in the metal layer 302a by removing the conductive material where it is not necessary (see Figure 5b).

Likewise, in a step 2006, a second substrate 300b is provided, uniformly deposited on which is a second layer of metal material 302b (see Figure 5c), and in a step 2008, via a wet-etching process, at least one solenoid is provided in the metal layer 302b (see Figure 5d).

In various embodiments, the two substrates 300a and 300b are then fixed together in a step 2010. For example, as shown in Figure 5e, the substrates 300a and 300b can be fixed together on the rear side, i.e., on the side without the metal layer. For instance, the two substrates 300a and 300b may be glued.

In various embodiments, the terminal parts at the centre of the solenoids are then electrically connected together in a step 2012 (see Figure 5f) via a metal contact 306, for example via soldering, rivets, etc., and the procedure terminates in a step 2014.

Figure 5g shows an embodiment in which the two substrates 300a and 300b are fixed together by positioning the first substrate 300a directly on the metal layer 300b. Also in this case, the terminal parts at the centre of the solenoids are electrically connected together. However, the embodiment shown with reference to Figure 5f presents the advantage that the connection between the two solenoids can be obtained in an easier way.

Consequently, in general, a solenoid is obtained, which is made up, in actual fact, of two solenoids 302a and 302b set on top of one another that are connected together in the internal area of the respective solenoids.

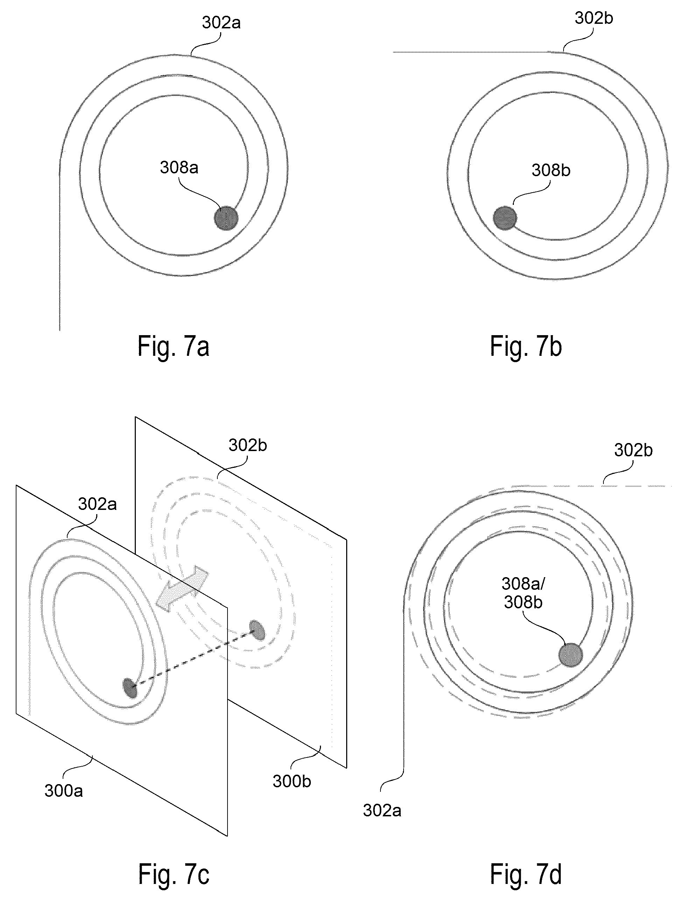

For example, Figure 7 shows an embodiment that makes it possible to obtain a substantially uniform electromagnetic field and to improve the number of turns.

In particular, in the embodiment considered, the solenoid obtained with the metallization 302a on the substrate 300a comprises n turns (Figure 7a).

In the embodiment, also the solenoid obtained with the metallization 302b on the substrate 300b comprises substantially the same number of turns, i.e., n turns. In particular, in various embodiments, these solenoids have the same width as that of the electrical paths that form the solenoid and are set apart by the same distance as the distance between the electrical paths.

For example, Figure 7b shows an embodiment of the solenoid 302b on the substrate 300b that is identical to the solenoid provided with the metallization 302a on the substrate 300a, with the only difference that the solenoid is rotated through 90° in a clockwise direction.

Next, as shown in Figure 7c, the two substrates 300a and 300b are connected together, and the electrical contact 306 is formed that connects the terminal portion 308a of the electrical path at the centre of the first solenoid 302a to the terminal portion 308b of the electrical path at the centre of the second solenoid 302b in such a way as to form a solenoid with twice the number of turns, i.e., 2n.

In particular, in the embodiment considered, the two solenoids are connected together on the side of the substrate, and consequently the solenoid 302b is shown from beneath and is reproduced specularly in Figure 7c.

In various embodiments, the terminals 308a and 308b may be widened as compared to the thickness of the paths of the solenoids in such a way as to facilitate connection between the solenoids 302a and 302b.

Consequently, in the embodiment considered, the solenoids 302a and 302b are identical, but the second solenoid 302b may be rotated with respect to the first solenoid 302a. For example, in the embodiment considered, the second solenoid 302b is rotated through 90° with respect to the first solenoid 302a.

This type of rotation should be taken into consideration also for the design of the connection terminals 308a and 308b.

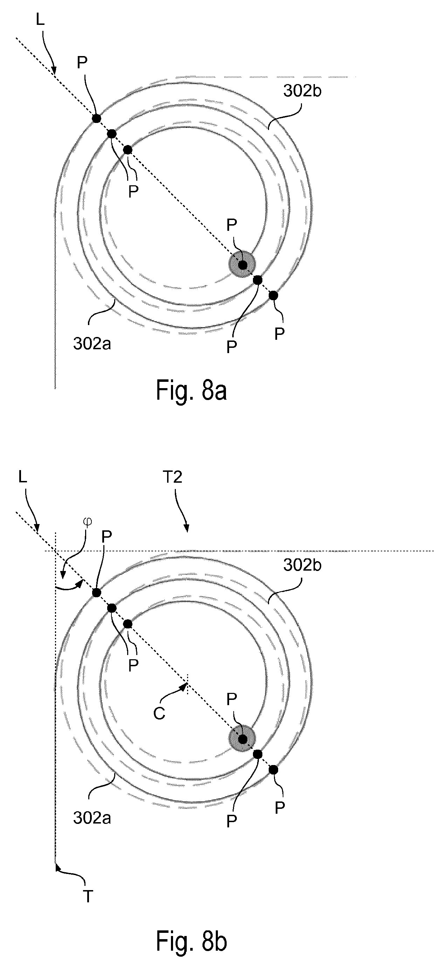

In various embodiments, the connection terminals 308a and 308b may be provided in one of the points P in which the metal paths 302a and 302b of the solenoids cross, when the two substrates 300a and 300b are connected together, i.e., in the points P where the spiral planes with circular shape that form the solenoids cross (see Figure 8a).

In particular, these points are typically set along a straight line L that passes through the centre C of the solenoid. In fact, in the case where the solenoids are identical, this line L is set on the axis of symmetry of the two solenoids set on top of one another. In particular, the axis of symmetry is half way between the tangent T1 of the external connection path of the first solenoid 302a and the tangent T2 of the external connection path of the second solenoid 302b (see Figure 8b). For example, in the embodiment considered, the two tangents T1 and T2 cross one another at an angle of 90°, and the angle $\varphi$ corresponds to 45°.

Instead, in the case where the second solenoid is not rotated, the tangents T1 and T2 do not cross, but the axis of symmetry L is in any case half way between the tangents T1 and T2 and corresponds to a line that is parallel to the tangents T1 and T2.

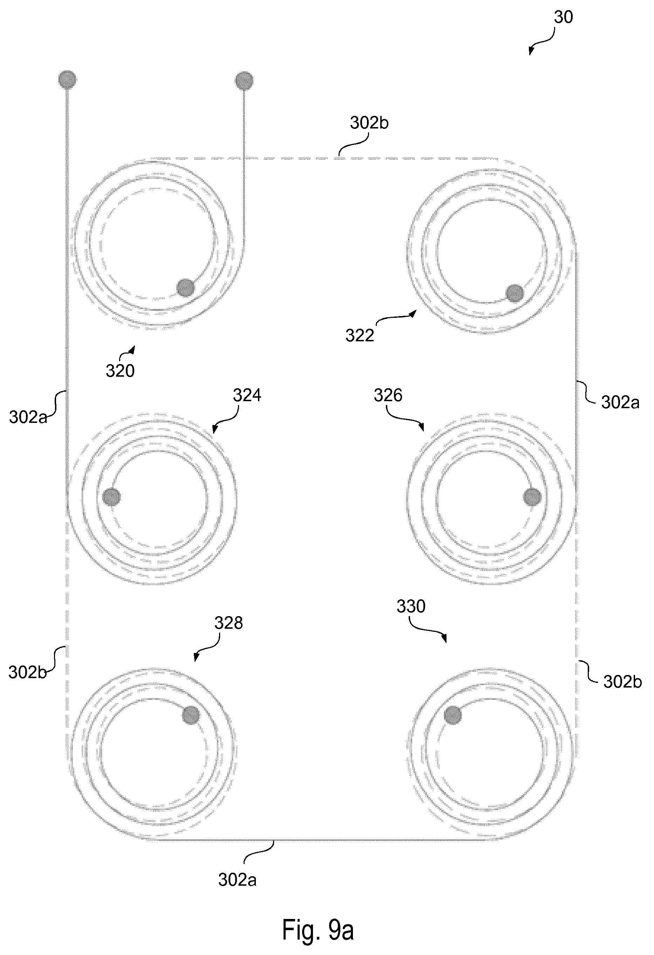

In general, the same type of connection can be used also for obtaining diffusers 30 that comprise a plurality of solenoids on one and the same substrate. For example, Figure 9a shows an embodiment of a diffuser 30 that comprises a plurality of solenoids that have been obtained on one and the same substrate and that may even have a number of turns different from one another.

In particular, in the embodiment considered, the diffuser 30 is provided via two substrates comprising, respectively, six solenoids (320, 322, 324, 326, 328, 330) that are connected together and are provided with the metal paths 302a and 302b arranged, respectively, on the two substrates.

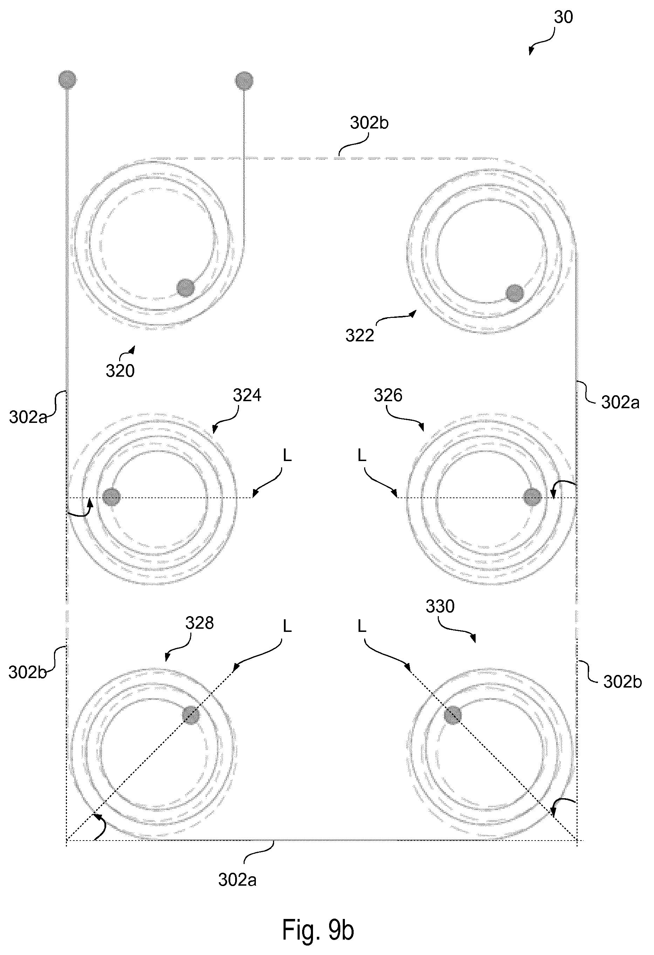

In particular, as shown in Figure 9b, in the embodiment considered, the solenoids 324, 326, 328 and 330 that are provided with the metal paths 302a and 302b are identical to one another and:

- the axis of symmetry L is at an angle $\varphi$ of 90° with respect to the tangents T1/T2 for the solenoid 324;
- the axis of symmetry L is at an angle $\varphi$ of 90° with respect to the tangents T1/T2 for the solenoid 326;
- the axis of symmetry L is at an angle $\varphi$ of 45° with respect to the tangents T1/T2 for the solenoid 328; and
- the axis of symmetry L is at an angle $\varphi$ of 45° with respect to the tangents T1/T2 for the solenoid 330.

However, in general, the two solenoids may even not be identical. For instance, the two solenoids 320 and 322 are not identical and, for example, have a number of turns slightly different, and consequently there does not exist an axis of symmetry.

In various embodiments, the diffuser, i.e., the two substrates that are fixed together, is then coated with a protective material, for example to guarantee impermeability and/or electrical insulation. For example, in one embodiment, each face of the diffuser 30 is coated with a silicone sheet.

In various embodiments, also other electrical components may be integrated in the diffuser. These components may be formed directly during the production process and/or soldered on appropriate metal contacts that are formed during the process.

For instance, in this way, strain/pressure sensors may be integrated for enabling detection of the effective presence of a patient on the diffuser. Moreover, electronic switches may be provided that are designed to enable or disable selectively one or more solenoids of the diffuser and/or deflect the current flow. In this way, stimulation areas may be selectively activated and/or different solenoids may be connected to different supply sources for selective turning-on.

As mentioned previously, magnetotherapy requires the use of solenoids of contained dimensions to be able to supply a magnetic field focused in the treatment area.

For instance, a typical solenoid could have a diameter of approximately $d_{ext}$ = 280 mm, and should be able to emit a peak magnetic field of 200 $\mu$T approximately, with a maximum current of 1.8 A.

In the example considered, the solenoid should be obtained from a sheet that comprises an aluminium layer with a uniform thickness h = 44 $\mu$m.

Moreover, it is assumed that the distance between the metal paths must be at least s = 1 mm. For example, this distance guarantees an appropriate electrical insulation between the paths and moreover frequently this value is imposed by the chemical-etching process. However, according to the supply voltage, even a smaller distance may be sufficient, for example 0.5 mm. In general, to reduce the diameter of the diffuser, it is also preferable for the distance s not to be excessive, and for this reason it is preferable for the distance s to be uniform and between 0.5 and 5 mm, preferably between 0.8 and 2 mm.

Using the Ampere-Laplace law, it may be deduced that, to obtain the required magnetic field, the solenoid should have approximately 36 turns.

Applying the superposition technique, the individual solenoids can have n = 18 turns. In this way, imposing the limit on the diameter of the solenoid referred to above, $d_{ext}$ = 28 cm, the width of the metal paths of the turns may be approximately $w$ = 6 mm. In fact, adding the distance s = 1 mm between the turns, the remaining internal diameter of the solenoid will be

$$\texttt{approximately } d_{int} = 42 \texttt{ mm, i.e.}$$

$$d_{int} = d_{ext} - 2 \cdot (w + s) \cdot (n - 1)$$

In general, once the thickness of the metal layer has been fixed, the width of the paths depends upon the maximum current. For instance, for typical applications of therapeutic treatment with electromagnetic waves, the width of the paths is uniform and should be between 1 and 10 mm, and preferably between 3 and 7 mm.

Consequently, the length of a single solenoid

$$\sum_{i=0}^{n-1} \pi \cdot [d_{ext} - 2 \cdot i \cdot (w + s)]$$

will be approximately $l$ = 9.104 m.

The electrical resistance R can be obtained by applying the second Ohm's law:

$$\rho \frac{l}{w} = 0.9655\Omega$$

where the resistivity of aluminium is

$$\rho = 0.028 \frac{\Omega \cdot mm^2}{m}$$

The two solenoids are connected, as described previously, in series so that the overall resistance is twice as much and equal to 1.931 $\Omega$.

Consequently, the intensity of the magnetic field emitted by the "double solenoid" is obtained with the following formula:

$$B = 2 \cdot \sum_{i=0}^{n-1} \frac{\mu_0 \cdot I}{\pi \cdot [d_{ext} - 2 \cdot i \cdot (w + s)]} = 2.158 G$$

i.e., approximately 216 $\mu$T, where the vacuum magnetic permeability is

$$\mu_0 = 4\pi 10^{-7} H/m$$

Instead, with the traditional technique, the solenoids should have a considerably greater size to achieve a comparable magnetic field. However, such a diameter would be frequently too large, in particular in the case of wearable diffusers, which may only have a maximum dimension.

Of course, without prejudice to the principle of the invention, the details of construction and the embodiments may vary widely with respect to what has been described and illustrated purely by way of example herein, without thereby departing from the scope of the present invention, as defined in the ensuing claims.

**Claims**

1. A production method of a diffuser (30) comprising the steps of:

- providing (2002, 2006) a first (300a) and a second (300b) substrate, wherein a respective uniform metallic layer (302a, 302b) is arranged on said first (300a) and said second (300b) substrate;
- implementing (2004) a first solenoid on said first substrate (300a) by means of an erosion process (1004-1012) of the respective metallic layer in an electrolytic bath, wherein said first solenoid comprises an external connection terminal and an internal connection terminal (308a);
- implementing (2008) a second solenoid on said second substrate (300a) by means of an erosion process (1004-1012) of the respective metallic layer in an electrolytic bath, wherein said second solenoid comprises an external connection terminal and an internal connection terminal (308b);
- fixing (2012) said first substrate (300a) to said second substrate (300a); and
- implementing an electrical connection between the internal connection terminal (308a) of said first solenoid and the internal connection terminal (308b) of said second solenoid.

2. Method according to Claim 1, wherein said first (300a) and said second (300b) substrate are in a plastic material, such as polyethylene or polyester.

3. Method according to Claim 1 or Claim 2, wherein the uniform metallic layer (302a, 302b) is in cooper or preferably in aluminum.

4. Method according to any of the previous claims, wherein the thickness of said uniform metallic layer (302a, 302b) is greater than 30 $\mu$m, preferably between 40 and 80 $\mu$m, preferably between 40 and 60 $\mu$m.

5. Method according to any of the previous claims, wherein la distance between the metallic tracks of said first and said second solenoid is between 0,5 and 5 mm, preferably between 0.8 and 2 mm.

6. Method according to any of the previous claims, wherein the width of the metallic tracks of said first and said second solenoid is between 1 and 10 mm, preferably between 3 and 7 mm.

7. Method according to any of the previous claims, wherein said first and said second solenoid have substantially the same number of turns.

8. Method according to Claim 7, wherein said first and said second solenoid are identically, but possibly arranged with different rotation angles.

9. Method according to any of the previous claims, comprising implementing a plurality of solenoids (320-330) on said first substrate (300a) and a plurality of solenoids (320-330) on said second substrate (300a).

10. A diffuser (30), preferably obtained with a production method according to any of the previous claims, wherein said diffuser (30) comprises:

- a first (300a) and a second (300b) substrate,
- a first solenoid in a metallic material with uniform thickness, wherein said first solenoid is arranged on said first substrate, and wherein said first solenoid comprises an external connection terminal and an internal connection terminal (308a);
- a second solenoid in a metallic material with uniform thickness, wherein said second solenoid is arranged on said second substrate, and wherein said second solenoid comprises an external connection terminal and an internal connection terminal (308b),

wherein said first substrate (300a) is fixed to said second substrate (300a), and wherein the internal connection terminal (308a) of said first solenoid is connected electrically to the internal connection terminal (308b) of said second solenoid.

Fig. 1

Fig. 2

a)

302

300

b)

304

302

300

c)

304a    304b

304

302

300

d)

304a

304

302

300

e)

304

302

300

f)

302

300

Fig. 3

Fig. 4

Fig. 5

Fig. 6

Fig. 7a

Fig. 7b

Fig. 7c

Fig. 7d

L

P

302b

P

P

302a

P

P

Fig. 8a

L

T2

φ

P

302b

P

C

P

302a

P

P

T

Fig. 8b

Fig. 9a

30

302b

320

322

324    326

302a

L    L

302a

328    L    L    330

302b    302b

302a

Fig. 9b

Europäisches Patentamt
European Patent Office
Office européen des brevets

# EUROPEAN SEARCH REPORT

Application Number

EP 16 17 8722

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br>Y | EP 2 533 255 A1 (F & B INTERNAT S R L [IT]) 12 December 2012 (2012-12-12)<br>* figures 1-3 *<br>* paragraph [0002] *<br>* paragraph [0022] - paragraph [0024] *<br>* paragraph [0036] - paragraph [0044] *<br>----- | 1-8,10<br><br>9 | INV.<br>H01F17/00<br>A61N2/02<br>A61N1/40 |
| Y,D | WO 2012/172504 A1 (THERESON S P A [IT]; CETRONI BRUNO MASSIMO [IT]) 20 December 2012 (2012-12-20)<br>* abstract; figure 7 *<br>----- | 9 | |
| X | US 2012/029343 A1 (WASSON JAMES R [US] ET AL) 2 February 2012 (2012-02-02)<br>* abstract; figures 1-5 *<br>* paragraph [0001] - paragraph [0009] *<br>* paragraph [0030] *<br>* paragraph [0036] - paragraph [0047] *<br>----- | 1-3,7,8,10 | |

TECHNICAL FIELDS SEARCHED (IPC)

H01F
A61N

The present search report has been drawn up for all claims

| Place of search | Date of completion of the search | Examiner |
|---|---|---|
| Munich | 28 November 2016 | Tano, Valeria |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 16 17 8722

This annex lists the patent family members relating to the patent documents cited in the above-mentioned European search report.
The members are as contained in the European Patent Office EDP file on
The European Patent Office is in no way liable for these particulars which are merely given for the purpose of information.

28-11-2016

| Patent document cited in search report | | | Publication date | Patent family member(s) | | | Publication date |
|---|---|---|---|---|---|---|---|
| EP 2533255 | A1 | | 12-12-2012 | EP<br>ES<br>PT | 2533255<br>2460923<br>2533255 | A1<br>T3<br>E | 12-12-2012<br>16-05-2014<br>07-05-2014 |
| WO 2012172504 | A1 | | 20-12-2012 | CA<br>CN<br>EP<br>US<br>WO | 2832713<br>103764223<br>2720752<br>2014187851<br>2012172504 | A1<br>A<br>A1<br>A1<br>A1 | 20-12-2012<br>30-04-2014<br>23-04-2014<br>03-07-2014<br>20-12-2012 |
| US 2012029343 | A1 | | 02-02-2012 | US<br>WO | 2012029343<br>2012015502 | A1<br>A2 | 02-02-2012<br>02-02-2012 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2012172504 A

- WO IB2015054797 W